Europäisches Patentamt

(19) European Patent Office

Office européen des brevets

(11) Publication number: **0 156 478**
**A2**

(12) **EUROPEAN PATENT APPLICATION**

(21) Application number: 85300933.0

(22) Date of filing: 12.02.85

(51) Int. Cl.⁴: **C 12 N 7/00**
**C 12 N 5/00, A 61 K 39/235**

(30) Priority: 13.02.84 US 579642

(43) Date of publication of application:
02.10.85 Bulletin 85/40

(84) Designated Contracting States:
AT BE CH DE FR GB IT LI LU NL SE

(71) Applicant: Veterinary Infectious Disease Organization
124 Veterinary Road
Saskatoon Saskatchewan, S7N 0W0(CA)

(72) Inventor: van den Hurk, Jan V.J.M.
235 Penryn Crescent
Saskatoon Saskatchewan S7H 5G5(CA)

(74) Representative: Griffin, Kenneth David et al,
Saunders & Dolleymore 2 Norfolk Road
Rickmansworth Hertfordshire WD3 1JH(GB)

(54) Propagation of group II avian adeno (AA) virus in avian cell culture and vaccine produced.

(57) A primary leukocyte cell culture and leukocyte cell line of avian origin have been found to be useful for the *in vitro* propagation of Group II avian adeno (AA) viruses (Haemorrhagic Enteritis Virus of turkeys, Marble Spleen Disease Virus of pheasants and Splenomegaly Virus of chickens). Propagation of the Group II AA viruses in leukocyte cell culture is useful in the production of a vaccine for protecting fowl against these same viruses.

EP 0 156 478 A2

-1-

# PROPAGATION OF GROUP II AVIAN ADENO (AA) VIRUS IN AVIAN CELL CULTURE AND VACCINE PRODUCED

1)      Field of the Invention

This invention deals with the prevention of Group II avian adeno (AA) virus related diseases in various avian species including turkeys, chickens and pheasants.  Further, this invention deals with vaccines for Group II AA viruses (Haemorrhagic Enteritis Virus of turkeys, Marble Spleen Disease Virus of pheasants and Splenomegaly Virus of chickens), their preparation and use.  A vaccine against diseases caused by Group II AA viruses is prepared in the present cell culture system.

## BACKGROUND OF THE INVENTION

Avian adenoviruses have been classified on the basis of serology as belonging to either Group I or Group II.  (Domermuth, C.H., et al, Avian adenovirus Group II.  The Haemorrhagic Enteritis - Marble Spleen Disease Viruses, presented at the 49th Northeastern Conference on Avian Diseases, University of Guelph, Guelph, Ontario 1977; and McFerran, J.B. Adenoviruses of Vertebrate Animals.  Chapter 3 in Comparative Diagnosis of Viral Diseases Vol. III. E. Kurstak and C. Kurstak (Eds).  Academic Press, Toronto, 1981, pgs. 102-165.)  Adenoviruses related to chicken embryolethal orphan virus (CELO) by common precipitating antigens are Group I avian adenoviruses, of which there appear to be at least 12 serotypes.  Group II avian adenoviruses include the causes of Haemorrhagic Enteritis of turkeys, Marble Spleen Disease of pheasants, and non-Marek's Disease Associated Splenomegaly (Marble Spleen Disease) of chickens.  Group II avian adeno (AA) viruses are therefore economically important diseases of poultry and account for annual losses in the poultry industry of millions of dollars. On the basis of an agar gel precipitin test there is no cross-reaction between viruses in Groups I and II however, this is a rather insensitive test system, hence the possibility of some serological relationship among viruses in the two groups cannot definitely be disregarded.

This invention relates to a successful procedure for the in vitro propagation of Group II AA viruses and to the application of this procedure to the production of Group II AA vaccines effective in the prevention of Group II AA related diseases.

2) Description of the Prior Art

As reported by C.H. Domermuth et al., (Vaccination for Haemorrhagic Enteritis of Turkeys. Avian Diseases, 21: 557-565, (1977); Vaccination of Ring-Necked Pheasant for Marble Spleen Disease. Avian Diseases 23: 30-38 (1979)), and J. Thorsen et al, (Field Trials of an Immunization Procedure Against Haemorrhagic Enteritis of Turkeys Avian Diseases 26: 473-477), Haemorrhagic Enteritis of Turkeys and Marble Spleen Disease of pheasants can be prevented by vaccination with avirulent strains of the virus.

For convenience, the vaccine virus is usually administered orally to young birds in the drinking water, however, other routes of inoculation, such as aerosolization or intravenous or intramuscular injection may be suitable. Until recently, it was not possible to propagate Group II AA viruses in cell culture systems or in avian embryos. Because of this, vaccine virus had to be produced by infecting turkey poults, killing them a few days later, removing their spleens, and preparing a crude homogenate of spleen tissue which contained sufficient virus to induce protection when fed to other birds.

There are several disadvantages associated with this system of vaccine production. Firstly, it is cumbersome in that it requires a large number of disease-free birds raised in isolation. Secondly, contamination of the vaccine with extraneous agents, such as Erysipelas species or other viruses, can also occur. Thirdly, it is difficult to standardize the potency of the vaccine because there is no simple method of determining the amount of virus in each batch. Finally, the vaccine is unstable because of the presence of tissue enzymes and hence must be stored at -70°C. Procedures for purifying the virus from crude spleen homogenates have been reported, however, these techniques do not totally overcome the disadvantages outlined above.

Fasina et al., (In Vitro Studies of Haemorrhagic Enteritis Virus with Immunofluorescent Antibody Technique. Avian Disease 26: 150-157 (1982)) demonstrated the in vitro infection of chicken, turkey and pheasant cells with HEV, however all attempts to passage the virus in these avian cell cultures have failed. Absent is a method for the continued in vitro progagation of the vaccine virus, and the use of spleen homogenates from infected birds for producing a vaccine has been the only method available for inducing immunity. Recently, Nazerian et al., (Establishment of B-Lymphoblastoid cell lines from Marek's Disease Virus-induced Tumors in Turkeys. International Journal of Cancer 29: 63-68, (1982)) Liu et al, (Establishment of Lymphoblastoid Cell Lines from Marek's Disease Primary Tumors, Poultry Science 62: 1902-1905 (1983)) have established lymphoblastoid cell lines from tumors of turkeys infected with Marek's Disease (MD) virus, an Herpes virus. Further, U.S. patent 4,388,298, Nazerian et al., describes the method of growing haemorrhagic enteritis virus (HEV) in a Lymphoblastoid cell line of turkey origin designated MDTC-RP-19. However, this is a transformed cell line which is also latently infected with Marek's Disease Virus. Hence, it may not be suitable for vaccine production.

Perrin et al., (Haemorrhagic Enteritis of Turkeys: Culture of Virus In Vitro, Bull. Acad. Vet. de France 54: 231-235 (1981)) outlines a preliminary study in which a spleen cell culture system is utilized for the in vitro replication of HEV/MSD virus. Thus, this experimentation by Perrin et al. suggests that HEV/MSD virus replicates in a cell system which consists mostly of white blood cells, however, their results appear to be inconclusive. In addition, this work fails to establish the possible effects of several passages in vitro and makes no attempt to incorporate the virus into vaccine.

Hence, previous attempts to culture Group II avian adeno (AA) virus, have except for the case of Nazerian mentioned above, been unsuccessful. There may be several explanations for this. Selection of the appropriate cell types which are susceptible to infection by Group II AA viruses is critical to the ultimate success in the propagation of these viruses in vitro. In previous experimentation for example, chicken and turkey fibroblast cultures or chicken and turkey kidney fibroblast cultures were utilized. These cultures did not support growth of the virus. Leukocytes, however, are the target cells in naturally infected birds. This natural susceptibility to infection in the bird was the basis for the selection of this cell type.

Another aspect which is critical to the success of the experimentation is the ability to distinguish between infected and non-infected cells. Through the development of a monoclonal antibody which reacts specifically with HEV and other viruses of the Group II AA virus group, an extremely sensitive fluorescent antibody test was established. This test allows for the detection of extremely low levels of virus. It is suspected that other investigators were not able to distinguish between infected and non-infected cells because they used the HEV antiserum produced in turkeys. This results in a great deal of background or non-specific fluorescence which reduces the sensitivity and specifity of the test considerably. Additionally, other investigators, notably Perrin et al, when testing for the presence of infectious virus in cell culture, have tended to examine the cell culture supernatant which contains a high level of residual virus from the original inoculum. Generally, greater than 90% of adenoviruses remain intracellular, and therefore it is believed that for this reason examination of the cells and cell extracts give a more accurate indication of the propagation of the infectious virus in cell culture.

Another aspect which enhances the success of the experimentation is the use of serum free of antibodies to Group II AA as a growth promotant in the culture medium. Previously, most investigators have used an agar gel precipitation test to detect the presence of antibodies in the serum. This test, while specific, is not very sensitive. Through the development of an extremely sensitive enzyme linked immunosorbent assay (ELISA) technique for measuring HEV antibody in serum, detection of much lower levels of antibody in serum can be achieved. This allows for a very accurate screening of the turkey serum prior to incorporating it into the culture medium. Since the sera of most turkeys, and probably chickens as well, contain antibodies to Group II AA viruses, it is likely that other investigators who incorporated serum into their culture media, even though it was screened by the agar gel precipitation test, may have been incorporating antibodies at the same time. These antibodies are capable of neutralizing the virus and may have prevented or reduced the degree of infectivity.

3)        Summary of the Invention

A method for the in vitro propagation of Group II avian adeno (AA) viruses is accomplished by a process of isolating leukocytes which are susceptible to infection with Group II AA viruses from body tissues or fluids (referred to hereandafter generally as tissues) of birds and growing them in culture. Subsequently, the cells are infected with Group II AA virus and, after a period of replication within the cells, the virus is harvested from the leukocyte cell culture. This method is useful in the propagation of both virulent and avirulent strains of Group II AA viruses and in the preparation of vaccines effective against haemorrhagic enteritis (HE) of turkeys, Marble Spleen Disease (MSD) of pheasants, non-Marek's Disease associated splenomegaly of chickens (SV) and other related diseases caused by Group II AA viruses in given avian species.

One aspect of the invention provides a method for the in vitro propagation of Group II avian adeno (AA) viruses comprising

a) isolating leukocytes harvested from the spleen, blood, bone marrow or other tissues of avian species, said leukocytes being susceptible to infection with said Group II AA viruses; and

b) culturing these leukocytes in a suitable growth medium; and

c) inoculating the leukocyte cell culture with one of the Group II AA viruses; and

d) propagating said inoculated cell culture under conditions suitable for the growth of the virus.

Another aspect of the invention is a method of establishing a cell line which will support the growth of Group II AA viruses comprising harvesting leukocytes from the tissues of avian species and serially passaging said leukocytes in culture.

Another aspect of the invention provides a method for the preparation of a vaccine active against HE, MSD, SV and other related diseases caused by avian adenoviruses in given avian species, which comprises in vitro propagation of the Group II AA virus in leukocyte cell culture thereby to produce a virus which can be used to induce immunity in said given species.

This invention provides a method for the preparation of a vaccine active against haemorrhagic enteritis of turkeys, marble spleen disease of pheasants, non-Marek's disease associated splenomegaly of chickens and other related diseases in poultry caused by Group II avian adeno (AA) viruses, whereby an in vitro propagated preparation of a Group II AA virus is utilized, this latter preparation comprising:

a) isolating leukocytes harvested from the spleen, blood, bone marrow or other tissues of avian species, said leukocytes being susceptible to infection with Group II AA virus; and

b) culturing these leukocytes in a suitable growth medium; and

c) inoculating the leukocyte cell culture with the Group II AA virus; and

d) propagating said inoculated cell culture under conditions suitable for the growth of said virus; and

e) recovering said virus.

Still another aspect of the invention is a vaccine for use in the immunization of turkeys and other avian species against haemorrhagic enteritis of turkeys, marble spleen disease of pheasants, non-Marek's disease associated splenomegaly of chickens, and other related diseases caused by Group II avian adeno (AA) viruses, comprising a viral component, said viral component being chosen from the group consisting of live virus, killed or inactivated virus, viral subunits or viral subcellular components, said component being recovered from in vitro propagation of Group II AA virus in a culture of leukocyte cells, wherein said cells have been isolated from the tissues of said avian species.

It is therefore an object of the present invention to provide a system of culturing Group II AA virus which is suitable for propagating virus in cells not latently infected with Marek's Disease Virus.

It is also an object of the invention to provide a safe, stable, and efficacious vaccine for immunizing turkeys and other avian species against diseases caused by Group II AA viruses.

Another object of the invention is to establish a leukocyte cell line which will support the growth of Group II AA viruses.

Other objects and advantages of the invention will become readily apparent from the ensuing description.

4)     Detailed Description of the Invention

The invention relates to a method of isolating leukocytes which are susceptible to infection with Group II AA viruses and culturing them. Preferably, leukocytes are harvested from the spleen, blood, or other tissues of turkeys, chickens (fowl), pheasants, or other species of birds using conventional cell separation techniques known in the art.

According to the invention, one preferred method of isolating the leukocytes from spleen comprises the steps of:

a) Euthanizing turkey poults, removing their spleens aseptically, and stripping the capsule from the spleens.

b) Homogenizing the spleen tissue in growth medium. (Tables 1 to 4).

c) Filtering the spleen homogenate through gauze to remove large cell clumps.

d) Concentrating and selectively isolating the leukocytes, for example by centrifuging through Ficoll-Paque* (trademark Pharmacia Fine Chemicals, Piscataway, New Jersey) solution for 20 minutes at 800 x g.

e) Harvesting the leukocytes from on top of the Ficoll-Paque, washing them twice in growth medium, and resuspending them in growth medium (approximately $5 \times 10^5 - 5 \times 10^7$ cells/ml).

f) Culturing the leukocytes in tissue culture flasks or dishes in an incubator in an atmosphere of about 2-7% (5%) $CO_2$, 90-99% (95%) relative humidity, and 37-43°C (41). Cells can be kept in culture and sub-cultured under these

*Trademark

conditions for up to 6 months. (Figures represent a suitable range; figures in brackets represent the optimum condition as far as can be determined at the present time.)

Table 1

| Component | Percent by Volume in Growth Medium |
| --- | --- |
| RPMI 1640 containing 25 mM Hepes buffer and gentamycin (50 mg/l) | 36 |
| McCoy's 5a | 13 |
| Leibovitz's L15 | 26 |
| Turkey serum | 10 |
| Fetal bovine serum | 10 |
| Tryptose phosphate broth | 5 |
| | 100 |

-11-

Table 2

RPMI 1640 MEDIUM[i]

| Component | mg/L |
|---|---|
| INORGANIC SALTS: | |
| $Ca(NO_3)_2.4H_2O$ | 100.00 |
| KCl | 400.00 |
| $MgSO_4$(anhyd.) | 48.84 |
| NaCl | 6,000.00 |
| $NaHCO_3$ | 2,000.00 |
| $Na_2HPO_4$(anhyd.) | 800.00 |
| | |
| OTHER COMPONENTS: | |
| D-Glucose | 2,000.00 |
| Glutathione (reduced) | 1.00 |
| Phenol red | 5.00 |
| | |
| AMINO ACIDS: | |
| L-Arginine (free base) | 200.00 |
| L-Asparagine | 50.00 |
| L-Aspartic acid | 20.00 |
| L-Cystine 2HCl | 65.15 |
| L-Glutamic acid | 20.00 |
| L-Glutamine | 300.00 |
| Glycine | 10.00 |
| L-Histidine (free base) | 15.00 |
| L-Hydroxyproline | 20.00 |
| L-Isoleucine (Allo free) | 50.00 |
| L-Leucine (Methionine free) | 50.00 |
| L-Lysine HCl | 40.00 |
| L-Methionine | 15.00 |
| L-Phenylalanine | 15.00 |
| L-Proline (Hydroxy L-Proline free) | 20.00 |
| L-Serine | 30.00 |

Continued.../

Table 2 (Continued)

| Component | mg/L |
|---|---|
| AMINO ACIDS (Continued): | |
| L-Threonine (Allo free) | 20.00 |
| L-Tryptophan | 5.00 |
| L-Tyrosine (Disodium Salt) | 28.83 |
| L-Valine | 20.00 |
| | |
| VITAMINS: | |
| Biotin | 0.20 |
| D-Ca pantothenate | 0.25 |
| Choline chloride | 3.00 |
| Folic acid | 1.00 |
| i-Inositol | 35.00 |
| Nicotinamide | 1.00 |
| Para-aminobenzoic acid | 1.00 |
| Pyridoxine HCl | 1.00 |
| Riboflavin | 0.20 |
| Thiamine HCl | 1.00 |
| Vitamin $B_{12}$ | 0.005 |

[1] Moore, G.E., Gerner, R.E., and Franklin, H.A. 1967. "Culture of Normal Human Leukocytes", J.A.M.A., 199: 519-524.

-13-

Table 3
McCOY'S 5a MEDIUM (modified)[1,2,3]

| Component | mg/L |
|---|---|
| **INORGANIC SALTS:** | |
| CaCl$_2$(anhyd.) | 100.00 |
| KCl | 400.00 |
| MgSO$_4$ . 7H$_2$O | 200.00 |
| NaCl | 6,460.00 |
| NaHCO$_3$ | 2,200.00 |
| NaH$_2$PO$_4$ . H$_2$O | 580.00 |
| | |
| **OTHER COMPONENTS:** | |
| Bacto-peptone | 600.00 |
| D-Glucose | 3,000.00 |
| Glutathione (reduced) | 0.50 |
| Phenol red | 10.00 |
| | |
| **AMINO ACIDS:** | |
| L-Alanine | 13.90 |
| L-Arginine HCl | 42.10 |
| L-Asparagine | 45.00 |
| L-Aspartic acid | 19.97 |
| L-Cysteine | 31.50 |
| L-Glutamic acid | 22.10 |
| L-Glutamine | 219.20 |
| Glycine | 7.50 |
| L-Histidine HCl . H$_2$O | 20.96 |
| L-Hydroxyproline | 19.70 |
| L-Isoleucine | 39.36 |
| L-Leucine | 39.36 |
| L-Lysine HCl | 36.50 |
| L-Methionine | 14.90 |
| L-Phenylalanine | 16.50 |

Table 3 (Continued)

| Component | mg/L |
|---|---|
| AMINO ACIDS (Continued): | |
| L-Proline | 17.30 |
| L-Serine | 26.30 |
| L-Threonine | 17.90 |
| L-Tryptophan | 3.10 |
| L-Tyrosine | 18.10 |
| L-Valine | 17.60 |
| | |
| VITAMINS: | |
| Ascorbic acid | 0.50 |
| Biotin | 0.20 |
| Choline chloride | 5.00 |
| D-Ca pantothenate | 0.20 |
| Folic acid | 10.00 |
| i-Inositol | 36.00 |
| Nicotinamide | 0.50 |
| Nicotinc acid | 0.50 |
| Para-aminobenzoic acid | 1.00 |
| Pyridoxal HCl | 0.50 |
| Pyridoxine HCl | 0.50 |
| Riboflavin | 0.20 |
| Thiamine HCl | 0.20 |
| Vitamin $B_{12}$ | 2.00 |

[1] McCoy, T.A. Maxwell, M. and Kruse, P.F., Proc. Soc. Exper. Biol. & Med., 100: 115-118 (1959).

[2] Hus, T.C. and Kellogg, D.S., Jr., J. Nat'l. Cancer Inst., 25: 221 (1960).

[3] Iwakata, S., Grace, J.T., Jr., N.Y. Jour. of Med., 64/No. 18: 2279-2282 (Sept. 15, 1946).

-15-

## Table 4
### L-15 (LEIBOVITZ) MEDIUM[1]

| Component | mg/L |
|---|---|
| **INORGANIC SALTS:** | |
| $CaCl_2$ (anhyd.) | 140.00 |
| KCl | 400.00 |
| $KH_2PO_4$ | 60.00 |
| $MgCl_2 \cdot 6H_2O$ | 200.00 |
| $MgSO_4 \cdot 7H_2O$ | 200.00 |
| NaCl | 8,000.00 |
| $Na_2HPO_4 \cdot 7H_2O$ | 359.00 |
| **OTHER COMPONENTS:** | |
| D (+) Galactose | 900.00 |
| Phenol red | 10.00 |
| Sodium pyruvate | 550.00 |
| **AMINO ACIDS:** | |
| DL-Alanine | 450.00 |
| L-Arginine (free base) | 500.00 |
| L-Asparagine | 250.00 |
| L-Cysteine (free base) | 120.00 |
| L-Glutamine | 300.00 |
| Glycine | 200.00 |
| L-Histidine (free base) | 250.00 |
| DL-Isoleucine | 250.00 |
| L-Leucine | 125.00 |
| L-Lysine (free base) | 75.00 |
| DL-Methionine | 150.00 |
| DL-Phenylalanine | 250.00 |
| L-Serine | 200.00 |
| DL-Threonine | 600.00 |
| L-Tryptophan | 20.00 |

Continued.../

-16-

Table 4 (Continued)

| Component | mg/L |
|---|---|
| AMINO ACIDS (Continued): | |
| L-Tyrosine | 300.00 |
| DL-Valine | 200.00 |
| | |
| VITAMINS: | |
| DL-Ca pantothenate | 1.00 |
| Choline Chloride | 1.00 |
| Folic acid | 1.00 |
| i-Inositol | 2.00 |
| Nicotinamide | 1.00 |
| Pyridoxine HCl | 1.00 |
| Riboflavin-5'-phosphate, sodium | 0.10 |
| Thiamine monophosphate | 1.00 |

[1] Leibovitz, Albert, Am. J. Hyg., 78: 173-180 (1963).

Another preferred method of isolating leukocytes from blood includes the following steps:

a) Drawing an equal volume of turkey blood into a syringe containing buffered anti-coagulant (for example phosphate buffered saline containing 0.005 mM sodium EDTA and 10 units/ml of heparin pH 7.2).

b) Layering this mixture on Ficoll-Paque and centrifuging for 20 minutes at 800 x g.

c) Harvesting the leukocytes from on top of the Ficoll-Paque, washing twice and resuspending the cells in growth medium.

d) Culturing the leukocytes as in (f) above.

e) After harvesting as in c) above, leukocytes from on top of the Ficoll-Paque can be further purified by centrifugation in a step-wise Percoll (30%, 40%, 50%, 60%, 70% Percoll in RPMI 1640) gradient for 30 minutes at 3,000 RPM. Cells on the surface of the 40% and 50% percoll solution are removed, washed twice in growth medium, resuspended to a concentration of approximately $10^6$ cells/ml and cultured under conditions described above.

The haemorrhagic enteritis virus (HEV) used to infect cell cultures included both virulent and avirulent strains. These strains were received from Dr. Charles Domermuth, Virginia Agricultural Experiment Station, Virginia Polytechnic Institute and State University, Blacksburg, Virginia in 1980. One preferred strain of HEV is the avirulent strain of the virus which is believed to be the same as the marble spleen disease (MSD) virus deposited in the American Type Culture Collection in Rockville, MD., and assigned Accession No. ATCC VR-898. Its origin and known characteristics have been previously

described in Domermuth, C.H. et al, J. Wild Dis 11: 77-74, 1975. For the purposes of cultivating Group II AA viruses in accordance with the invention, either this HEV/MSD virus or any other Group II AA virus having similar characteristics would be useful. This HEV/MSD culture is known not to cross react with other Group I avian adenoviruses, but does cross react with other Group II AA viruses.

Other sources of virus for inoculating the leukocyte culture can include, but is not restricted to, body fluids (such as blood), tissues (such as spleen), or feces of birds infected with Group II AA virus. In one preferred embodiment, crude virus-containing extract, or extrct purified by chloroform extraction and cesium chloride centrifugation, from lymphatic organs, preferably spleen, are prepared from turkeys infected with virulent or avirulent Group II AA virus as follows:

a) Turkeys infected with Group II AA virus are sacrificed 4-6 days after infection, their spleens removed and added to 9 volumes of hypotonic Tris-buffer (0.01 mM Tris HCl pH 8.1).

b) Spleens are homogenized (for example using the Brinkman polytron for 10-15 minutes).

c) Homogenate is treated to release virus from the spleen cells as for example by freezing and thawing twice and/or sonicating (3 times for 1 minute at 400 watts with a Braunsonic 1510).

d) Centrifuging (10,000 x g for 10 minutes) to remove cellular debris, and using the virus containing supernatant to inoculate leukocyte cell cultures.

The crude extract described above can be further purified using the following steps starting at d).

e) Combining the crude extract with chloroform in a ratio of 1:2 (v/v) and repeating the homogenization, then centrifuging for 10 minutes at 10,000 RPM in order to separate the extract into 3 layers: a top aqueous phase containing the virus, an intermediate layer containing cellular debris, and a bottom chloroform layer containing fatty material.

f) The clear aqueous phase (top layer) is harvested and the virus pelleted overnight at 10,000 RPM, resuspended in Tris-buffer and extracted an additional 2 or 3 times in chloroform.

g) Following the final extraction, the virus is concentrated by pelleting for 1 hour at 20,000 RPM using a cesium chloride cushion, and then banded twice through a cesium chloride gradient for 24 hours at 40,000 RPM. The majority of infectious virus is found in a band having a density of 1.33-1.34 $g/c^3$.

h) The harvested virus is dialized against storage buffer, filtered through a 0.45 $\mu$ (micron) Millipore filter and used to inoculate leukocyte cell cultures, or stored at -70°C.

Either virulent or avirulent strains of Group II AA viruses can be cultured in the cells. These virus strains differ in their pathogenicity for birds. For example, avirulent strains of HEV cause only transient splenomegaly while virulent strains cause splenomegaly as well as intestinal bleeding and death in some cases.

Cell cultures are infected with virus-containing extracts as outlined above, usually within 1-3 hours after the cell culture is established. An appropriate dilution of virus-containing extract, usually in the range of $10^{-2}$ to $10^{-6}$ depending on the infectivity of each virus preparation, is added to the cell culture. Infected cell cultures are maintained in growth medium and incubated at 41°C in an atmosphere of 5% $CO_2$ and 95% RH. Generally the virus is harvested 1-3 days after inoculation. The infected cells in the cultures are mostly enlarged and contain intranuclear inclusions which can be observed with a light microscope. There is no difference in appearance between leukocyte cultures infected with a virulent or avirulent strain of HEV.

The presence of viral antigens in the leukocytes after infection is detected by a fluorescent antibody (FA) test, or by other tests known in the art, using mouse monoclonal antibodies against specific proteins of Group II AA viruses. In this test, leukocytes from cell culture are fixed onto glass slides by techniques commonly used in the art. The fixed cells are incubated for 1 hour at 37°C in a humid chamber in hybridoma cell medium. After washing the cells, fluorescein-labelled goat anti-mouse IgG is added and the cells are incubated for 1 hour at 37°C after which time the preparations are mounted using techniques known in the art and observed under a fluorescence microscope. Cells infected with Group II AA virus fluoresce and the proportion of infected cells is estimated by counting fluorescent cells and total cells in several microscope fields. The infected cells are usually 10-12 µ in diameter, sometimes larger. They often contain vacuoles in the cytoplasm and show nuclear inclusion bodies which are seen in both adhered and non-adhered cells.

Ultra-thin sections of fixed leukocytes cultures examined under the electron microscope revealed virus particles (70-90 nm) in the nuclei of the cells. The cells were quite large (10-40 µ), had phagosomes and mitochondria in the cytoplasm and perinuclear chromatin in the nucleus. Based on these observations it is felt that the cells are immature large macrophages or monocytes. However, this cannot be confirmed simply on the basis of morphology.

The invention further relates to the preparation of a vaccine for the immunization of turkeys and other avian species against HEV of turkeys, MSD of pheasants and non-Marek's disease associated splenomegaly of chickens and other related diseases associated by Group II AA viruses.

In preparing a vaccine for use in inoculating birds against HEV, the virus can be recovered from the infected whole cell culture, cells harvested from the whole cell culture, the culture medium or the supernatant fluid, by techniques known in the art. For example, adhered cells are detached from the tissue culture flask with a rubber policeman and together with non-adhered cells are pelleted by centrifugation (5 minutes at 2,000 RPM). The cells are disrupted to promote virus release by suspending them in hypotonic Tris-buffer (0.01 mM Tris HCl, pH 8.1), freezing and thawing twice, and/or sonicating (for example, 3 cycles of 20 seconds at 100 watts, using the Braunsonic 1510 sonicator). Cellular debris is removed by centrifugation (10 minutes at 10,000 RPM), the supernatant containing the virus is recovered and dialized against storage buffer, filtered through a 0.45 µ filter, combined with storage buffer and stored.

One preferred method for the recovery of the virus comprises a) rupturing the leukocyte cells by sonication, freezing and thawing, or osmotic shock and b) selectively separating the virus containing the supernatant from cellular debris by centrifugation. The resultant supernatant is then dialized and combined with a preferred storage buffer, the formula of which is 0.02 M Tris-HCl, 0.001 M MgCl$_2$, 5% (volume/volume) glycerol, and 5% (weight/volume) bovine serum albumin; pH 8.0.

This buffer is a combination of 2 buffers previously reported in the literature. The sources are: 1) Prage, L., Petterson, U., Hoglund, S., and Lonberg-Holm, K., and Philipson, L., Structural Proteins of Adenoviruses. (IVC Sequential Degradation of the Adenovirus Type II Viron. Virology 42, 341-358, 1970). In this reference they outline the following buffer: 0.25 M sucrose, 0.02 M Tris, 0.002 M MgCl$_2$ and 0.5% butanol; pH 7.4. The second reference is: Cepko, C.L., Changelian, P.S. and Sharp, P.A., ("Immunoprecipitation with Two-Dimensional Pools as a Hybridoma Screening Technique: Production and Characterization of Monoclonal Antibodies Against Adenovirus II Proteins. Virology 110, 385-401. 1981). The buffer outlined is 0.02 M Tris, 0.001 M MgCl$_2$ and 5% glycerol; pH 8.0.

The preferred buffer combines portions of each of these two and in addition includes 5% bovine serum albumen. The use of a cell culture grown virus plus the use of a buffer provides a stable product which can be stored in a liquid form. This is an advantage and an improvement over the currently used crude spleen extract vaccine which, because of the presence of tissue enzymes, is unstable and must be stored at -70°C. Other buffers known to the art may also be used to dialyze and combine with the vaccine for storage. The vaccine can then also be lyophilized for storage.

Furthermore, the vaccine may be combined with an adjuvant selected from the group of Freund's Adjuvant, aluminum hydroxide, aluminum potassium sulfate, or other suitable adjuvants known in the art.

It is envisioned that vaccinal amounts of vaccine would be administered orally in the drinking water or feed, by aerosol, or by other conventional methods to protect against HE in turkeys, MSD in pheasants and SV of chickens. Insofar as the avirulent HEV is known to protect fowl against virulent HEV, it of course would be the preferred strain for use in vaccine production. However, other avirulent Group II AA would also be suitable.

Also provided by the present invention are various criteria for determining suitable ranges for defining an effective turkey dose.

a) Although at the present time it is difficult to quantify the number of virus particles in the culture harvest, selection of a culture harvest containing a minimum of 10 virus particles is believed to provide a safe and effective dose.

b) Culture harvest containing sufficient virus to induce a serum antibody titer of at least 1/20 as determined by ELISA. At the present time, effective protection can be achieved with an antibody titer of 1/80. However, since this may vary somewhat with the age of the bird, a more conservative titer has been selected to define a protective dose.

An indirect ELISA for the titration of HEV antibodies was carried out in 3 steps: (i) HEV, used for coating was extracted from spleens of experimentally infected turkeys and purified on CsCl gradients. The virus band with a density of 1.34 $g/c^3$ was treated with an equal volume of 2 M $CaCl_2$ and incubated for 30 min at 37°C. Thereafter, 96 well polystyrene microtiter plates (Immulon *2, Dynatech Laboratories) were coated with antigen (0.2 ml per well) in an optimal dilution of 0.05 M sodium bicarbonate buffer, pH 9.6, for 2h at 37°C. After coating the plates, they were washed three times with PBS containing 0.05% Tween 20* (PBS-T), pH 7.4, (ii) serum samples to be tested and positive and negative control sera in two-fold dilutions in 10 x PBS-T containing 1% (V/V) newborn calf serum were incubated for 2h at 37°C (0.2 ml per well), (iii) after three washings with PBS-T, the plates were incubated with 0.2 ml rabbit anti-turkey Ig G-peroxidase conjugate (diluted 1:1000) (containing 1% newborn calf serum) for 2h at 37°C. After three more washings with PBS-T, the plates were incubated with 0.2 ml per well of substrate solution containing 5-aminosalicyclic acid (0.08%, W/V) and $H_2O_2$ (0.005% V/V), pH 6.0. The enzymatic activity was measured after 30 min at room temperature with a micro-ELISA reader (MR 580, Dynatech Laboratories) at 492 nm. All tests were done in duplicate and each assay included 2 positive control sera (with known titers) and 4 negative control sera.

c) Culture harvest used at dilutions ranging from undiluted to $10^{-9}$, but preferably at a range of $10^{-2}$ to $10^{-4}$.

A further preferred embodiment of the present invention is the establishment of a leukocyte cell line which will support the growth of Group II AA viruses and comprises harvesting leukocytes from the tissues of avian species, which includes but is not limited to the spleen, blood and bone marrow, and serially passaging the leukocytes in culture.

The following examples are intended only to further illustrate the invention and are not intended *Trademark to limit the scope of the invention which is defined by the claims.

EXAMPLE 1 - INFECTION OF TURKEY SPLEEN LEUKOCYTES WITH AVIRULENT HAEMORRHAGIC ENTERITIS VIRUS

Leukocyte cultures were prepared from the spleens of 5 and 12 week old turkey poults. The leukocytes were obtained from homogenized decapsulated turkey spleens and isolated by centrifugation onto a Ficoll-Paque solution for 20 min at 800 x g. The leukocytes were subsequently harvested, washed, resuspended in growth medium (1 x $10^6$ cells per ml), plated in tissue culture flasks and incubated in an atmosphere of 5% $CO_2$, 95% relative humidity and 41°C. Three hours after seeding, the cell cultures were inoculated with crude spleen extract containing avirulent HEV or purified virus prepared as described above. One, 2 and 3 days after inoculation with virus, the cell cultures were examined by FAT to determine the percent of infected cells (Tables 5 and 6).

Table 5 - Growth of Avirulent Haemorrhagic Enteritis
Virus in Leukocyte Cell Culture Prepared
from Spleens of 5-Week Old Turkeys

| Source of virus inoculum | Dilution | % infected cells on day[a] | | |
|---|---|---|---|---|
| | | 1 | 2 | 3 |
| Crude spleen extract | $10^{-2}$ | 1.0 | 1.4 | 1.8 |
| | $10^{-3}$ | 2.7 | 0.7 | 1.0 |
| Purified virus | $10^{-2}$ | 0.4 | 0.3 | 0.7 |
| | $10^{-3}$ | $< 0.1$ | $< 0.1$ | $< 0.1$ |
| Virus-free control (PBS) | -- | $< 0.1$ | $< 0.1$ | $< 0.1$ |

Table 6 - Growth of Avirulent Haemorrhagic Enteritis
Virus in Leukocyte Cell Culture Prepared
from Spleens of 12-Week Old Turkeys

| Source of virus inoculum | Dilution | % infected cells on day[a] | | |
|---|---|---|---|---|
| | | 1 | 2 | 3 |
| Crude spleen extract | $10^{-3}$ | 2.4 | 3.2 | 1.6 |
| | $10^{-4}$ | 1.5 | 1.2 | 3.2 |
| Purified virus | $10^{-3}$ | 0.8 | 0.8 | 1.6 |
| | $10^{-4}$ | $< 0.1$ | $< 0.1$ | $< 0.1$ |
| Virus-free control (PBS) | -- | $< 0.1$ | $< 0.1$ | $< 0.1$ |

[a] Percent of cells which were infected with HEV-A
on various days after infection as determination
by FAT.

-27-

EXAMPLE 2 - INFECTION OF TURKEY BLOOD LEUKOCYTES WITH AVIRULENT HAEMORRHAGIC ENTERITIS VIRUS

Leukocyte cultures were prepared from blood of 9 week old turkeys. An equal volume of turkey blood was drawn into a syringe containing anticoagulant solution (0.005 N sodium-EDTA and 10 units of heparin per ml in RPMI 1640 medium). The blood cells were separated by centrifugation through a Ficoll-Paque solution for 20 min at 800 x g. The leukocyte fraction was collected, washed, resuspended in growth medium, whereafter the cells were seeded in tissue culture flasks (2 x $10^{-6}$ cells per ml) and incubated in an atmosphere of 5% $CO_2$, 95% relative humidity and 41°C. On days 0, 1, 3 and 7 after being established, the leukocytes were infected with a crude spleen extract containing avirulent HEV. Cultures were examined at various times after infection to determine the percentage of infected cells (Table 7).

Table 7 - Infection of Turkey Blood Leukocyte Cultures at Various Times
After Preparation with Avirulent Haemorrhagic Enteritis Virus

| Day Cell Culture was infected | Percent cells infected on day after inoculation with virus | | | | | | | | | | |
|---|---|---|---|---|---|---|---|---|---|---|---|
| | 1 | 2 | 3 | 4 | 5 | 6 | 7 | 8 | 9 | 10 | 11 |
| 0 | 1.6[a] | 1.9 | 0.5 | NE[c] | $<$0.1 | NE------------------------------------------- | | | | | |
| | 0.2[b] | 1.0 | 0.6 | NE | 2.3 | NE------------------------------------------- | | | | | |
| 1 | $<$0.1 | 0.5 | NE | 1.3 | NE | 0.0 | NE--------------------------------- | | | | |
| | $<$0.1 | $<$0.1 | NE | $<$0.1 | NE | 0.0 | NE--------------------------------- | | | | |
| 3 | NE | 0.4 | NE | 0.0 | 0.0 | NE | 0.0 | NE----------------------- | | | |
| | NE | 1.5 | NE | 0.0 | $<$0.1 | NE | 0.0 | NE----------------------- | | | |
| 7 | 0.0 | NE | 0.0 | 0.0 | NE----------------------------------------------- | | | | | | |
| | 0.0 | NE | 0.0 | 0.0 | NE----------------------------------------------- | | | | | | |

[a] Percent non-adhered cells infected with virus as determined by FAT.
[b] Percent adhered cells infected with virus as determined by FAT.
[c] NE - not examined.

-29-

EXAMPLE 3 - COMPARISON OF TURKEY BLOOD LEUKOCYTES
              OBTAINED BY TWO DIFFERENT PROCEDURES FOR
              THEIR ABILITY TO SUPPORT THE GROWTH
              OF HEV

Thirty ml of blood was obtained from a 9 week old turkey poult. Leukocytes were separated from the whole blood by either the Ficoll-Paque method or the Ficoll-Paque followed by the Percoll purification step, and used to establish cell cultures. The Ficoll-Paque purification was carried out as outlined in Example 2. The Percoll procedure included further separation of the leukocytes by centrifuging the leukocyte fraction obtained after Ficoll-Paque purification through a stepwise Percoll gradient (30, 40, 50, 60 and 70% Percoll in RPMI 1640) for 30 min at 3,000 RPM. The leukocytes on the surface of the 40 and 50% Percoll were collected, washed, resuspended in growth medium, seeded in tissue culture flasks and maintained in an environment of 5% $CO_2$, 95% relative humidity and 41°C. The cultures were infected with a $10^{-3}$ dilution of a crude spleen extract of avirulent HEV, and 2 and 4 days following inoculation the percentage of infected cells in each leukocyte culture was determined (Table 8).

-30-

Table 8 - Infection with Avirulent HEV of Turkey
Blood Leukocyte Cultures Separated by
Two Different Procedures

| | Percent infected cells on day[c] | |
|---|---|---|
| Method of preparing leukocyte culture | 2 | 4 |
| Ficoll-Paque | 1.9 | 0.5 |
| Ficoll-Paque + Percoll | 6.0 | 3.9 |

[c] Percent non-adhered cells infected as determined by FAT. The percentage of cells infected with virus-free control was $<0.1\%$.

EXAMPLE 4 - USE OF HEV GROWN IN LEUKOCYTE CELL
CULTURE AS A VACCINE AGAINST HAEMORRHAGIC
ENTERITIS OF TURKEYS

Thirty ml of blood collected from a 20 week-old turkey was used to prepare a leukocyte culture by the Ficoll-Paque method described above. The leukocyte cultures were infected with a $10^{-3}$ dilution of a crude spleen extract containing avirulent HEV. Two days after infection, 4.3% of the cells were infected as determined by the FA test. At this time, adhered and non-adhered cells were harvested by centrifugation. The virus was released from the cells by resuspending the cell pellet in hypotonic Tris buffer (0.01 M Tris-HCl, pH 8.1), freezing and thawing twice and sonicating. Following low speed centrifugation to remove debris, the virus-containing supernatant was combined with PBS to a total volume of 30 ml.

Serial 10-fold dilutions (undiluted to $10^{-4}$) of the virus-PBS solution were prepared. One ml of each dilution was used to orally vaccinate 6 week old turkey poults (8 turkeys/group). Serum samples, to determine antibody titers to HEV were taken on days 1, 8, 15 and 20 after vaccination. The turkeys were orally challenged by inoculation with virulent HEV on day 15. Five days following challenge with virulent virus (on day 20) the birds were sacrificed and the spleens collected and examined for the presence of enlargement and HEV antigen. HEV antigen, indicating lack of protection, was present in all 8 unvaccinated control birds after challenge. Only 8 of 39 vaccinated birds had HEV antigen indicating that the cell culture grown virus was protective (Table 9). In addition 31 out of the 39 birds seroconverted and developed protective antibody titers after vaccination.

Table 9 - Proportion of Turkeys Protected Against Experimental Challenge with Virulent HEV by Various Dilutions of Vaccine Produced in Leukocyte Cell Culture

| Vaccine dilution | No.of turkeys affected[a] Total No.in group | No.of turkeys with protective[b] antibody titer Total No.in group |
|---|---|---|
| $10^0$ | 0/8 | 8/8 |
| $10^{-1}$ | 0/8 | 8/8 |
| $10^{-2}$ | 0/8 | 8/8 |
| $10^{-3}$ | 2/8 | 6/8 |
| $10^{-4}$ | 6/7 | 1/7 |
| Non-vaccinated controls | 8/8 | 0/8 |
| Non-vaccinated, Non-challenged controls | 0/8 | 0/8 |

[a] As determined by the presence of HEV antigen in spleens by antigen-ELISA 5 days after challenge with virulent HEV.

[b] As determined with antibody-ELISA.

EXAMPLE 5 - PASSAGE OF AVIRULENT HEV IN PRIMARY
BLOOD LEUKOCYTE CULTURES

Crude spleen extract from avirulent HEV-infected turkeys (final dilution $10^{-3}$) was used for the initial infection of blood leukocytes from turkeys obtained by Ficoll-Paque centrifugation (passage 1). After 3 days in culture the cells were collected and virus was released from the cells by freezing and thawing twice and ultrasonic treatment as described above. HEV harvested from passage 1 was used to infect a new culture of turkey blood leukocytes (passage 2). These cells were processed as before to release virus which was subsequently used to infect new leukocytes (passage 3). This process was repeated for 7 passages of HEV in turkey blood leukocytes. At each step, 10% of the previous cell extract was used to infect the same number of leukocytes as in the previous culture (Table 10). The culture conditions for the cultures were the same as described before.

Table 10 - Passage of Avirulent HEV in Primary Blood Leukocyte Cultures

| Days Post-Infection | Percentage Infected Cells[a] | | | | | | |
|---|---|---|---|---|---|---|---|
| | Passage Number | | | | | | |
| | 1 | 2 | 3 | 4 | 5 | 6 | 7 |
| 1 | NE[b] | NE | <0.1 | NE | <0.1 | NE | NE |
| 2 | 4.3 | 1.8 | 0.6 | 0.3 | 0.4 | NE | 0.4 |
| 3 | NE | 3.8 | 1.0 | 0.6 | 0.9 | 1.8 | 1.65 |

[a] Determined by FAT.
[b] NE - not examined.

EXAMPLE 6 - THE ABILITY OF PASSAGED (SUBCULTURED)
TURKEY BLOOD LEUKOCYTES TO SUPPORT THE
GROWTH OF HEV

Turkey blood leukocytes were obtained by centrifugation of 50 ml blood on a Ficoll-Paque gradient as described earlier and maintained in culture dishes under the same conditions as described above. The cells of 1 culture dish were not subcultured while the cells of the 3 other culture dishes were subcultured 2 times, 3 times and 4 times, respectively thereby creating a leukocyte cell line. Both the primary leukocyte culture and leukocyte cell line were infected with crude spleen extract of avirulent HEV infected turkeys (final extract diluted $10^{-4}$). Data from this experiment are presented in Table 11.

Table 11 – Growth of Avirulent HEV in Primary Leukocyte Cell Culture
and a Leukocyte Cell Line

| Type of Cell Culture System | No.of Times Leukocytes Subcultured | Percentage of Infected Turkey Leukocytes[a] Days Post-Infection | | | | | | | | |
|---|---|---|---|---|---|---|---|---|---|---|
| | | 1 | 2 | 3 | 4 | 5 | 6 | 7 | 8 | 9 |
| Primary culture | 0 | <0.1 | 0.4 | 2.0 | 2.0 | 1.0 | 1.5 | NE[b] | 0.9 | NE |
| (Primary) cell line | 2 | <0.1 | 1.6 | 2.6 | 2.2 | 3.5 | 2.9 | NE | NE | <0.1 |
| (Primary) cell line | 3 | <0.1 | 1.7 | 2.6 | 3.5 | 2.2 | NE | 0.1 | NE | NE |
| (Primary) cell line | 4 | 0.3 | 1.1 | 1.5 | 0.4 | NE | NE | NE | NE | NE |

[a] Percentage of infected cells determined by FAT.

[b] NE – not examined.

EXAMPLE 7 - THE EFFICACY OF HEV SERIALLY PASSED IN CELL CULTURE AS A VACCINE FOR HE IN TURKEYS

Avirulent HEV serially passed seven times in leukocyte cell culture and harvested 3 days post-infection as outlined in Example 5 (Table 10) was used. The virus-containing suspension was combined with PBS to a total volume of 15 ml, whereafter its efficacy as a vaccine for HE in turkeys was tested in a dose response challenge trial as described in Example 4. All birds vaccinated at a dilution of $10^{-1}$, $10^{-2}$, $10^{-3}$, and 3 of 8 birds vaccinated at a dilution of $10^{-4}$, were found to be protected when challenged by the following criteria: 1) they had protective antibody titers $\geq 80$ by ELISA; 2) they did not have HEV antigen in their spleens (titers $< 100$ by antigen ELISA); and 3) they did not have intestinal haemorrhages. No protection was found after challenge in the remaining 5 birds vaccinated at a dilution of $10^{-4}$, and in the 8 control birds which were not vaccinated.

This experiment shows clearly that after seven serial passages of the avirulent strain of HEV in cell culture the produced virus still can be used as an effective vaccine.

Table 12 - Vaccine Trial Using Avirulent HEV
          Serially Passed 7 Times in Leukocyte
          Cell Culture

| Dilution of Culture Harvest Used to Vaccinate Poults | No.of turkeys affected[a] Total No.in Group | No.of turkeys with protective[b] antibody titer Total No.in Group |
|---|---|---|
| $10^{-1}$ | 0/8 | 8/8 |
| $10^{-2}$ | 0/8 | 8/8 |
| $10^{-3}$ | 0/8 | 8/8 |
| $10^{-4}$ | 5/8 | 3/8 |
| Non-vaccinated controls | 8/8 | 0/8 |

[a] As determined by the presence of HEV antigen in
   spleen by antigen-ELISA 5 days after challenge with
   virulent HEV.

[b] As determined with antibody-ELISA 5 days after
   challenge with virulent HEV.

EXAMPLE 8 - INFECTION OF CHICKEN AND TURKEY
            LEUKOCYTES WITH GROUP II AVIAN
            ADENOVIRUSES

    Leukocytes were obtained from turkey blood
or chicken blood by purification on Ficoll-Paque
gradients, and were maintained under conditions as
described previously. Leukocytes were infected with
crude spleen extract from: a) chickens infected with
the virus which causes non-Marek's Disease associated
with splenomegaly (SV) or, b) turkeys infected with
the avirulent strain of haemorrhagic enteritis virus
(HEV-A). The results are presented in Tables 13 and
14. Note that 4 out of 8 chicken leukocyte cultures
became infected with SV, and 8 out of 8 with HEV-A (Table 13). Of the turkey leukocyte cultures,
2 of 6 became infected with SV, and 6 of 6 with HEV-A
(Table 14).

Table 13 - Infection of Chicken Leukocytes with
Haemorrhagic Enteritis Virus (HEV-A) and
Splenomegaly Virus (SV)

| Leukocytes from chicken number | Cell cultures infected with Group II AA Virus | | | |
| | 3 days post infection | | 4 days post infection | |
| | HEV-A | SV | HEV-A | SV |
| --- | --- | --- | --- | --- |
| 152 | +[a] | + | + | + |
| 156 | + | + | + | - |
| 158 | + | - | + | - |
| 159 | + | - | - | - |
| 163 | -[b] | - | + | + |
| 164 | + | - | + | - |
| 169 | + | - | - | - |
| 170 | + | - | - | + |

+[a] = infected cells detected with the FA test.

-[b] = no infected cells were detected in these cell
cultures.

Table 14 - Infection of Turkey Leukocytes with
Haemorrhagic Enteritis Virus (HEV-A)
and Splenomegaly Virus (SV)

| | Cell cultures infected with Group II AA Virus | | | |
| | 3 days post-infection | | 4 days post-infection | |
| Leukocytes from turkey number | HEV-A | SV | HEV-A | SV |
|---|---|---|---|---|
| 101 | +[a] | −[b] | + | − |
| 102 | + | + | + | + |
| 103 | + | − | + | − |
| 104 | + | − | + | − |
| 107 | + | − | + | − |
| 108 | + | + | + | + |

+[a] = infected cells detected with the FA test.
−[b] = no infected cells were detected in cell cultures.

EXAMPLE 9 - COMPARISON OF THE PERCENTAGE OF INFECTED LEUKOCYTES USING AVIRULENT OR VIRULENT HEV AS INOCULUM

Leukocyte cultures were prepared from blood collected from an 18-week old turkey, separated by the Ficoll-Paque method, and thereafter maintained under conditions as described before. The leukocyte cultures were infected with dilutions of crude spleen extracts from turkeys either infected with avirulent HEV or virulent HEV. The percentage of infected cells was determined by the FA test (Table 15).

Table 15 - Comparison of the Percentage of Infected Leukocytes Using Avirulent HEV (HEV-A) or Virulent HEV (HEV-V) as Inoculum

| Source of Virus Inoculum | % infected cells 3 days post-infection[a] dilution of virus inoculum | | | | |
|---|---|---|---|---|---|
| | $10^{-3}$ | $10^{-4}$ | $10^{-5}$ | $10^{-6}$ | $10^{-7}$ |
| HEV-A[b] | 2.1 | 5.7 | 1.9 | 0.1 | < 0.1 |
| HEV-V[c] | 1.8 | 3.2 | 0.8 | < 0.1 | < 0.1 |

[a] Percent of the cells which were infected as determined by FAT.

[b] Crude spleen extract of HEV-A infected turkey.

[c] Crude spleen extract of HEV-V infected turkey.

-40-

We Claim:

1. A method for the _in vitro_ propagation of Group II avian adeno (AA) viruses comprising

(a) isolating leukocytes harvested from the spleen, blood, bone marrow or other tissues of avian species, said leukocytes being susceptible to infection with said Group II AA viruses; and

(b) culturing these leukocytes in a suitable growth medium; and

(c) inoculating the leukocyte cell culture with one of the Group II AA viruses; and

(d) propagating said inoculated cell culture under conditions suitable for the growth of the virus.

2. The method as described in claim 1 wherein said Group II avian adeno (AA) virus is haemorrhagic enteritis virus (HEV).

3. The method as described in claim 1 wherein said Group II avian adeno (AA) virus is Marble Spleen Disease virus of pheasants or the virus which causes non-Marek's disease associated splenomegaly of chickens.

4. The method as described in claim 1 wherein said virus is a virulent strain.

5. The method as described in claim 1 wherein said virus is an avirulent strain.

6. The method as described in claim 1 wherein said leukocyte cell culture is a primary leukocyte cell culture.

7. The method as described in claim 1 wherein said leukocyte cell culture is a leukocyte cell line culture.

8. A method of establishing a cell line which will support the growth of Group II avian adeno (AA) viruses comprising harvesting leukocytes from the tissues of avian species and passaging (subculturing) said leukocytes in culture.

9.     The method as described in claim 8 wherein said tissues are selected from spleen, blood and bone marrow.

10.     A method for the preparation of a vaccine active against haemorrhagic enteritis of turkeys, marble spleen disease of pheasants, non-Marek's disease associated splenomegaly of chickens and other related diseases caused by Group II avian adeno (AA) viruses in given avian species, which comprises in vitro propagation of the Group II AA virus in leukocyte cell culture thereby to produce a virus which can be used to induce immunity in said given species.

11.     A vaccine for use in the immunization of turkeys and other avian species against haemorrhagic enteritis of turkeys, marble spleen disease of pheasants, non-Marek's disease associated splenomegaly of chickens, and other related diseases caused by Group II avian adeno (AA) viruses, comprising a viral component, said viral component being chosen from the group consisting of live virus, killed or inactivated virus, viral subunits or viral subcellular components, said component being recovered from the in vitro propagation of Group II AA virus in a culture of leukocyte cells, wherein said cells have been isolated from the tissues of said avian species.

12.     The vaccine as described in claim 11 wherein the viral component is recovered from:

          the whole cell culture;

          cells harvested from the whole cell culture;

          the culture medium; or the supernatant fluid;

or other suitable fractions cntaining said virus.

13.     The vaccine as described in claim 11 wherein the vaccine is combined with a buffer.

14.     A vaccine as described in claim 11 wherein the vaccine is freeze-dried.

15.     A method for the preparation of a vaccine active against haemorrhagic enteritis of turkeys, marble spleen disease of pheasants, non-Marek's disease associated splenomegaly of chickens and other related diseases in poultry caused by Group II avian adeno (AA) viruses, whereby an in vitro propagated preparation of a Group II AA virus is utilized, this latter preparation comprising:

(a) isolating leukocytes harvested from the spleen, blood, bone marrow or other tissues of avian species, said leukocytes being susceptible to infection with Group II AA virus; and

(b) culturing these leukocytes in a suitable growth medium; and

(c) inoculating the leukocyte cell culture with the Group II AA virus; and

(d) propagating said inoculated cell culture under conditions suitable for the growth of said virus; and

(e) recovering said virus.

16.     A method as described in claim 15 wherein the recovery of said virus comprises

(a) rupturing the leukocyte cells by sonication, freezing and thawing or osmotic shock;

(b) selectively separating the virus containing supernatant from the cellular debris by centrifugation.

17.     A method as described in claim 16 wherein the resultant supernatant is dialized against a storage buffer, and then combined with said storage buffer.

18.    A vaccine as described in claim 11 wherein said viral component is present in sufficient quantity to induce a serum antibody titer of at least 1/20 as determined by enzyme linked immunosorbent assay (ELISA), to provide an effective dose.

19.    A vaccine as described in claim 11 in unit dosage form wherein said vaccine contains a minimum of 10 virus particles per unit.

20.    A vaccine as described in claim 11 wherein said vaccine is at a dilution of 0 to $10^{-9}$ to provide an effective dose.